# EUROPEAN PATENT APPLICATION

(11) **EP 4 356 831 A1**
(43) Date of publication of application: **24.04.2024**
(21) Application number: 23179313.4
(22) Date of filing: 14.06.2023
(51) Int. Cl.: A61B 5/145

(54) **IMPLANTABLE DEVICE**

(30) Priority: 19.10.2022 GB 202215447
(71) Applicant: Chordata Limited, Epsom KT17 3PU (GB)
(72) Inventor: McMahon, Alastair, Epsom, KT17 3PU (GB); Curtis, Peter, Epsom, KT17 3PU (GB); Wisbey, John, Epsom, KT17 3PU (GB)
(74) Representative: Gill Jennings & Every LLP

(57) **Abstract**

An implantable device for detecting biomarkers in animals, the device comprises: a sensor comprising: a pair of electrodes spaced apart from each other, the pair of electrodes having a first capacitance; an oscillator configured to oscillate at a first frequency and output a first signal at the first frequency; wherein the sensor is configured to receive an amount of biological fluid wherein the biological fluid causes the capacitance of the pair of electrodes to change from the first capacitance to a second capacitance; wherein the oscillator is coupled to the pair of electrodes and arranged such that a change in the capacitance of the pair of electrodes causes a change in oscillation of the oscillator from the first frequency to a second frequency; and wherein the oscillator is configured to output a second signal at the second frequency, the second signal indicative of a biomarker in the biological fluid.

## Description

### FIELD OF THE INVENTION

The present invention relates to an implantable device for implanting into animals such as livestock. In particular, the implantable device can be used to detect biomarkers in animals.

### BACKGROUND

It is important to monitor and track the health of animals, for example livestock, in order to ensure that they have a long lifespan and a high quality of life. This can be done by monitoring the animal's vital signs through data collection and taking appropriate action, for example any necessary medical intervention, when the collected data indicates there are anomalies.

One method of tracking vital signs in animals is to detect and monitor biomarkers which are present in biological fluid. Biomarkers are naturally occurring molecules, genes, or characteristics by which a particular pathological or physiological process, disease, etc. can be identified. Since each biomarker is indicative of a particular condition or disease, detection of a particular biomarker in the biological fluid of an animal indicates that the animal has the particular condition or disease associated with the detected biomarker.

One method of detecting biomarkers in biological fluid involves taking a sample of biological fluid from the animal and performing tests on the sample to determine whether any biomarkers are present.

However a problem with this approach is that in order for the animal to be monitored regularly, regular samples of biological fluid will need to be taken which is both invasive and distressing for the animal over time.

Another problem with this approach is that it generally requires the sample to be manually taken from the animal and so a medical person must be present when taking the sample, and as well during future sampling. This is time consuming and inefficient.

Furthermore, manually taking samples from an animal means that monitoring is intermittent rather than constant. As such, the animal may experience problems which go undetected for a period of time.

Biomarkers in livestock can also be detected using electrochemical sensors. Current electrochemical sensors used for biomarker detection can be grouped into potentiometric, amperometric, voltammetric, impedimetric, and conductometric sensors. In other words, current sensors detect changes in resistance, potential difference, current, or analyte concentration.

These detection methods, however, have several drawbacks including optimizing the stability, storage, and logistics of electrochemical sensors, as well as maintaining their optimum functionality in diverse samples that are barely treated or diluted.

The present invention aims to address at least some of these problems.

### SUMMARY OF INVENTION

According to a first aspect there is provided an implantable device for detecting biomarkers in animals, the device comprising: a sensor comprising: a pair of electrodes spaced apart from each other, the pair of electrodes having a first capacitance; an oscillator configured to oscillate at a first frequency and output a first signal at the first frequency; wherein the sensor is configured to receive an amount of biological fluid wherein the biological fluid causes the capacitance of the pair of electrodes to change from the first capacitance to a second capacitance; wherein the oscillator is coupled to the pair of electrodes and arranged such that a change in the capacitance of the pair of electrodes causes a change in oscillation of the oscillator from the first frequency to a second frequency; wherein the oscillator is configured to output a second signal at the second frequency, the second signal indicative of a biomarker in the biological fluid.

The biological fluid may be any biological fluid that contains biomarkers for example blood or interstitial fluid. The biomarkers may be detected in any suitable animal including livestock for example, but not limited to, canine and bovine livestock.

The sensor may also be referred to as a first sensor.

The implantable device is based on the dielectric spectroscopy principle for detecting the biomarkers biological fluid of animals. This provides an accurate way of determining the biomarkers present within the biological fluid because each biomarker is associated with an oscillation frequency which can be detected.

In some examples, the first frequency is in the range 10-100 GHz and more preferably in the range 10-30 GHz. In this frequency range, the dielectric constant of biomarkers typically present within biological fluid can be easily detected. In addition, this frequency range provides the most accurate results for dielectric sensing because undesired parameter-dependent dispersion mechanism of the biological cells, which exists in low-frequency ranges, has negligible effects on the sensor functionality and so the signal-to-noise ratio (SNR) is generally better.

Preferably, the sensor is a terahertz sensor. The terahertz sensor is a dielectric sensor preferably operating in the terahertz frequency regime (between 0.1 THz and above). The terahertz sensor preferably uses BiCMOS processes. BiCMOS combines the benefits of Bipolar and CMOS, and so BiCMOS is able to achieve circuits with speed-power-density performance which cannot be achieved by those technologies individually. Also BiCMOS circuits are much faster for large fan-out due to its small sensitivity to capacitive loading, which is advantageous for sensing aspects. Using a high-frequency, terahertz sensor allows the sensor to be embedded onto a small-sized chip, which is ideal for implanting as the overall size of the implantable device can be smaller. A terahertz sensor also provides high sensitivity to biomarker detection. Additional advantages of using a terahertz sensor may include excellent reproducibility, low noise, and low jitter.

In some examples, the pair of electrodes and oscillator may form an integrated circuit. Preferably the integrated circuit is an application specific integrated circuit (ASIC).

In some examples, the integrated circuit may comprise a III-V based Hetro-junction bipolar transistor.

The implantable device may further comprise a transponder configured to wirelessly communicate with an external device.

Preferably, the transponder is configured to transmit data corresponding to the second signal to the external device. The transponder may be configured to transmit data corresponding to the second signal to the external device in response to receiving an indication to transmit data.

The transponder may be configured to receive power transmitted by the external device so that the transponder can power the implantable device. In this way, the transponder may be a passive transponder.

Preferably, the sensor and transponder are encapsulated in a package. In some examples, the package comprises fan-out wafer-level packaging. In other examples, a laser direct structuring (LDS) process can be used, which facilitates easy integration of the RF antenna in the encapsulation. The transponder may comprise NFC and RF components. Thus, the integrated circuit, NFC, and RF components may be encapsulated within a single package. The use of fan-out wafer-level packaging allows a higher degree of integration to be obtained whilst providing a greater number of external contacts. It provides a smaller package footprint with higher input/output (I/O) along with improved thermal and electrical performance.

Preferably the package is housed within a housing. The housing may be an epoxy mould housing. The housing may ensure that the implantable device comprises a single component, which reduces the number of individual components that need to be implanted into the animal. Furthermore, a single component helps limit migration of individual components.

The housing may further comprise a biocompatible coating. The coating may be a bioglass coating for example parylene or diamond-like carbon (DLC). The coating may be configured to seal the package within the housing. Preferably, the coating may hermetically seal the package and its components within the housing, which may help ensure that the implantable device is safe to insert into an animal.

Preferably, the coating comprises at least one through-hole configured to allow biological fluid to contact the sensor. This provides a simple and convenient mechanism for allowing the biomarkers to be detected by the sensor. In some examples, the housing may comprise at least one through-hole configured to allow biological fluid to contact the sensor. In this case, the at least one through-hole in the housing and the at least one through-hole in the coating are substantially in alignment such that there can be considered effectively a single through-hole through the coating and the housing to the sensor, to allow the biological fluid to contact the sensor.

In some examples, the sensor may be integrated onto a first microchip and the transponder may be integrated onto a second microchip. In other examples, the sensor and transponder may be integrated onto a single microchip.

The implantable device provides significant advantages over current detectors in terms of its integration and operation in the terahertz regime, where ultrafast data transfer rate between the implantable device and the wearable device is accomplished. Current biomarker detection methods require a nanoparticle coating on the surface of the detecting electrodes. However the implantable device described herein uses a normal electrode and it is covered by a thin bioglass coating apart from the sensor region. This arrangement advantageously enables the efficient contact with biological fluids. The integrated system also provides a small overall footprint in a single package for an implantable device which is easily injectable.

In some examples the implantable device further comprises an additional sensor. The additional sensor may also be referred to as a second sensor. Preferably, the additional sensor is a near infrared sensor or mid-infrared sensor. The additional sensor may comprise a photodetector and an emitter. The emitter may be configured to emit a light at an amount of biological fluid and the photodetector may be configured to detect the light reflected and / or transmitted through the biological fluid in order to determine a biomarker present in the biological fluid.

The emitter may comprise at least one micro-LED, preferably a plurality of micro-LEDs.

The additional ("second") sensor is preferably configured to detect at least one biomarker that is not detectable by the ("first") sensor.

According to another aspect there is provided a system for monitoring the well-being of animals, comprising an implantable device as described above; and a wearable device configured to wirelessly communicate with the implantable device.

Preferably, the wearable device is further configured to power the implantable device by generating and transmitting an RF signal which can be received by the transponder of the implantable device, for example using one or more antenna.

In this way, the implantable device is passive and is powered by the wearable device. Advantageously, RF energy may be harvested from the wearable device to the implantable device which can power the sensor. Additionally, the sensor signal may be amplified and sent to an ADC (analogue to digital converter), which may send a digital signal to the wearable device.

The wearable device may comprise at least one motion detector configured to detect motion in order to determine a position of the wearable device. The wearable device may also comprise a memory configured to store data received from the implantable device.

According to another aspect there is provided a wearable device comprising: a receiving device configured to receive data from an implant in an animal, the data indicative of a biomarker present in biological fluid of the animal; a memory configured to store the data; and a power generating device configured to wirelessly transmit power to the implant. The implant is an implant according to any of the previously described implants.

The receiving device may comprise an RF reader. In some examples, the power generating device comprises an RF antenna.

According to another aspect there is provided a kit of parts comprising an implantable device according to any of the previously described implantable devices and a wearable device according to any of the previously described wearable devices, the wearable device configured to wirelessly communicate with the implantable device.

According to another aspect there is provided an implantable device for detecting biomarkers in animals, the device comprising: a sensor configured to receive an amount of biological fluid; wherein the sensor comprises: an emitter configured to emit radiation at the biological fluid; and a photodetector configured to detect radiation reflected or transmitted through the biological fluid; and wherein the detected radiation is indicative of a biomarker in the biological fluid.

It will be understood that the wearable device and implantable device can be used with any animal not limited to livestock. For example the wearable device and the implantable device can be used with animals that can be considered as companions, such as pets, including but not limited to cats and dogs.

The wearable device and implantable device can also be used with humans. In this case, the implantable device detects biomarkers in humans. The implantable device functions in the same way as described previously in relation to animals. However, for human uses, although the wearable device functions the same way, the wearable device may be modified for example the wearable device may be a bracelet or other wearable suitable to be worn by a human instead of a collar, although in some cases the wearable device may be a necklace which may be considered a form of collar.

It will be understood by a skilled person that any apparatus feature described herein may be provided as a method feature, and vice versa. It will also be understood that particular combinations of the various features described and defined in any aspects described herein can be implemented and/or supplied and/or used independently. Furthermore, as used herein, any "means plus function" features may be expressed alternatively in terms of their corresponding structure.

### BRIEF DESCRIPTION OF DRAWINGS

The present invention will be described by way of example only with reference to the accompanying drawings in which:
Figure 1 shows an exemplary implantable device and wearable device;
Figure 2 shows an exploded view of an exemplary implantable device;
Figure 2a shows a circuit forming part of an implantable device;
Figure 3 shows a schematic view of an exemplary implantable device and wearable device;
Figure 4 shows a cross sectional view of an exemplary implantable device;
Figure 5 shows part of a circuit forming part of an implantable device; and
Figure 6 shows a schematic view of an exemplary implantable device.

### DETAILED DESCRIPTION

The invention generally relates to an implantable device 2 for detecting biomarkers in animals 8, the implantable device 2 being implanted within the animal 8 to monitor their vital signs to increase their lifespan and quality of life. A wearable device 4, such as a collar, is worn by the animal 8 which wirelessly communicates with the implantable device 2, for example via a reader 26 in the wearable device 4. The implantable device 2 sends data relating to detected biomarkers to the wearable device 4 where the data is stored temporarily before being transferred to an external computing device.

Details of the implantable device 2, the wearable device 4, and their operation will now be described.

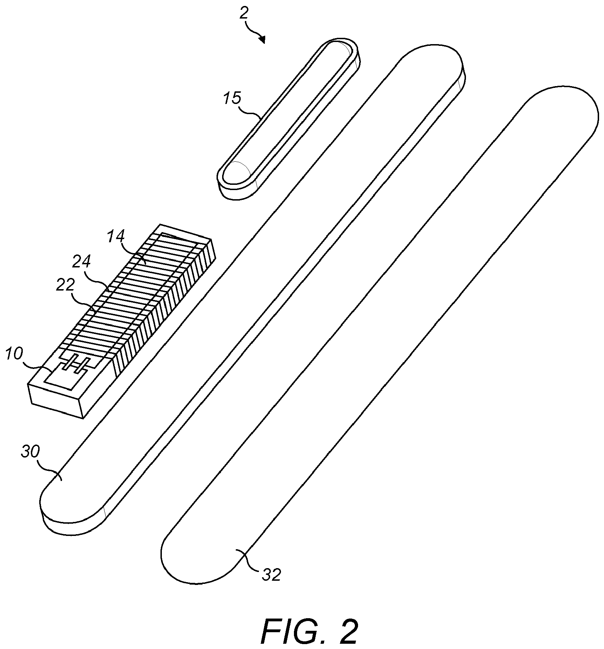

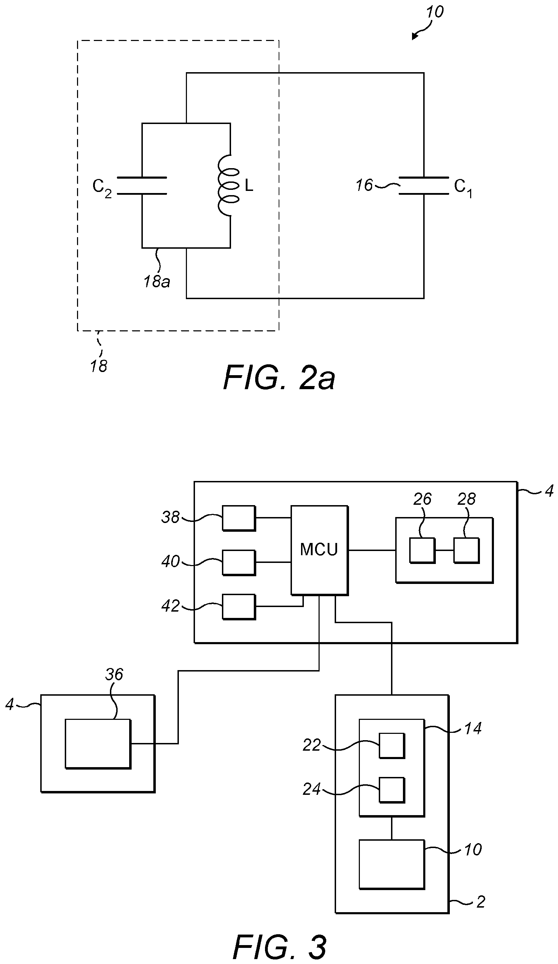

A shown in Figure 2a, the implantable device 2 comprises a sensor 10 formed by a sensing capacitor C₁ and an oscillator 18. The sensing capacitor C₁ comprises pair of electrodes 16 spaced apart from each other, the pair of electrodes 16 having a first capacitance. The oscillator 18 comprises a capacitor C₂ and an inductor L connected in parallel to form an LC resonant tank 18a, which generates a signal indicative of the oscillation frequency of the oscillator. The oscillator 18 is electrically connected in parallel to the sensing capacitor C₁. A capacitance change of the sensing capacitor C₁ affects the free oscillation frequency of the oscillator 18 which can be detected as a change in voltage.

The sensor 10 generally operates based on electro impedance spectrometry (EIS) where a high frequency signal (for example in the GHz range, preferably 100s of GHz range) is applied to electrodes and the electrical response of the chemical system is measured. In this case, the chemical system comprises the biological fluid of the animal. As discussed above, biological fluid is a rich source of biomarkers which can be used provide information about the health of an animal.

The sensor is configured to receive an amount of biological fluid comprising at least one biomarker. Each biomarker has its own dielectric constant and so when the biological fluid contacts the sensing capacitor C₁ of the sensor 10, in particular when the biological fluid enters the space between the pair of electrodes 16 of the sensing capacitor C₁, the capacitance of the sensing capacitor C₁ changes due to the change in dielectric constant as a result of the presence of a biomarker. In other words, the presence of biological fluid on the sensor 10 causes the capacitance of the pair of electrodes 16 to change from a first (i.e. initial) capacitance to a second capacitance.

The change in capacitance of the sensing capacitor C₁ causes a change in oscillation frequency of the oscillator 18 from a first frequency (i.e. the free oscillation frequency of the oscillator) to a second frequency. The change in oscillation frequency of the oscillator therefore depends on the dielectric constant of the biomarker and so each biomarker corresponds to a particular oscillation frequency of the oscillator 18. The oscillator 18 generates a signal at the oscillating frequency which can be detected and measured as a voltage, and so the detected and measured voltage can be used to determine what biomarkers are present in the biological fluid.

The different dielectric constants for different biomarkers are pre-determined through laboratory experiments and so the individual biomarker oscillation frequencies can be determined for each biomarker. This data can be saved in a database and accessed later. Since each change in measured voltage, from an initial voltage corresponding to the free oscillation frequency to a second voltage corresponding to the change in oscillation frequency, is as a result of the change in dielectric constant at the sensing capacitor C₁, the second measured voltage corresponds to a particular biomarker. In other words, the detected voltage of a particular oscillation frequency indicates the biomarker present in the biological fluid.

The dielectric constant, and therefore oscillation frequency, for various combinations of multiple different can also be calibrated through laboratory experiments so that combinations of biomarkers can also be detected, as well as detecting individual biomarkers. As an example, a biomarker combination of 10% glucose and 90% cortisol may correspond to an oscillation frequency of 17.5GHz. If this combination changes, for example to 10% cortisol and 90% glucose, the corresponding oscillation frequency will also change, for example to 23GHz. Different combinations of biomarkers are therefore calibrated using experiment data and simulations.

In summary, frequency changes of the oscillator 18, which can be detected as a change in voltage, indicate which biomarkers are present in the biological fluid, and so important information about the health of the animal can be determined.

The operating frequency of the sensor is chosen to be in the range of 10-100 GHz, preferably in the range 10-30 GHZ, where the dielectric constant of biomarkers can easily be detected. Furthermore, this frequency range provides the most accurate results for dielectric sensing of biomarkers in biological fluid because undesired parameter-dependent dispersion mechanisms of the biological cells, which exist in low-frequency ranges, have negligible effects on the sensor functionality and so the signal-to-noise ratio (SNR) is generally better. Typically, there will be a 5-10% change in oscillating frequency of the oscillator 18, depending on the particular dielectric constant change of the biomarker.

The sensor 10 takes the form of a terahertz dielectric sensor and the sensing capacitor C₁ is formed by interdigitated capacitor (IDC) electrodes underneath a dielectric layer. The sensing capacitor C₁ and oscillator 18 are embedded on a chip to form an integrated circuit, which is preferably an application specific integrated circuit (ASIC).

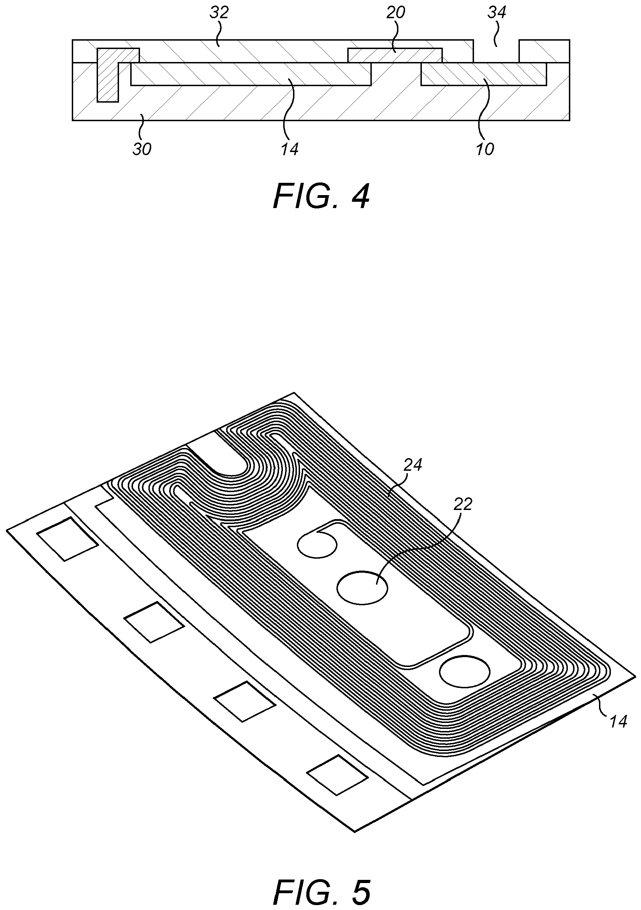

Turning to Figure 2, the implantable device 2 comprises a transponder 14, in particular a high-frequency (HF) RFID transponder. The transponder 14 includes transceiver 22, in the form of a NFC transceiver, and an implant antenna 24 also shown in Figure 3. The implant antenna 24 is an NFC antenna in the form of RF coils. The transponder 14 can be embedded on an NFC chip to form an integrated circuit. In this way, the sensor 10 is integrated onto a first chip and the transponder 14 is integrated onto a second chip. An exemplary chip comprising the transponder 14 is shown in Figure 5. The two chips can be connected together using a conductive link. In some exemplary implantable devices 2, the transponder 14 and the sensor 10 are integrated onto a single chip to form a single component.

The integrated circuit (preferably, an ASIC) formed by embedding the transponder 14 onto an NFC chip is electrically connected to the sensor 10 through an inductive link 20, for example as shown in Figure 4, this link 20 used to supply power to the sensor 10 by the ASIC. The power level provided depends on a number of different influencing factors including antenna size, distance to the reader unit, movement of the reader unit during transmission, duration, and frequency of the measurement. As mentioned above, in some examples, the sensor 10 is embedded onto the same chip as the transponder forming part of the integrated circuit (preferably the ASIC).

The ASIC is configured to convert the analogue output data from the sensor 10 to digital data which can be sent to the wearable device 4. The ASIC incorporates silicon germanium, or any III-V based Hetro-junction bipolar transistor (HBT), to drive the high-speed terahertz signal transfer between the implantable device 2 and the wearable device 4.

Using a high-frequency, terahertz sensor provides the advantage that the sensor 10 is embedded onto a small-sized chip, which is ideal for implanting as the overall size of the implantable device 2 can be smaller, as well as providing high sensitivity to biomarker detection. In addition, a high frequency sensor having a small-sized chip significantly reduces the biological sample volume requirements.

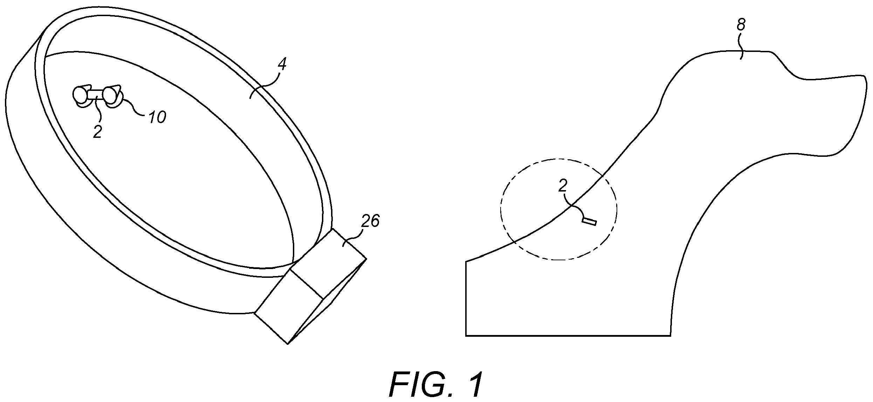

The transponder 14 is configured to wirelessly communicate with the wearable device 4 which comprises a reader 26 in the form of an RFID reader, and a wearable antenna 28, as shown in Figure 3. The wearable antenna 28 is an NFC antenna in the form of RF coils. The implantable device 2 and the wearable device 4 together form an RFID system, for example as shown generally in Figure 1. Using the RFID technology, the sensor 10 can be powered and read wirelessly by the reader 26, as will be described in more detail later.

The sensor 10 and transponder 14 are encapsulated in a single package using fan-out wafer-level packaging (FOWLP). This method of encapsulation allows a higher degree of integration to be obtained whilst providing a greater number of external contacts. Additionally FOWLP provides a smaller package footprint with higher input/output (I/O) along with improved thermal and electrical performance. Moreover a laser direct structuring process can also be used as an alternate to FOWLP, as it complements the integration of RFcoil on the package along with efficient energy harvesting. Further, it is also much easier to volume manufacture.

As shown in Figure 4, for example, the package is housed within a housing 30 which in some examples takes the form of an epoxy mould housing. Encapsulation and housing multiple components (namely the sensor 10 and the transponder 14) in a single package reduces the number of individual components that need to be implanted into the animal 8, simplifying the implant procedure. A single package also limits migration of components within the animal.

As further shown in Figures 2 and 4, the housing further comprises a coating 32, which is configured to seal the package within the housing 30. The coating 32 is a hermetic coating made of any suitable ISO-approved biocompatible material, for example bioglass and/or parylene / DLC (diamond like carbon). As such, the coating 32 hermetically seals the package and its components within the housing, ensuring that the implantable device 2 is safe to insert into an animal.

In order to allow a sample of biological fluid to come into contact with the sensor 10, the coating 32 comprises at least one through-hole 34, as shown in Figure 4. The through-hole 34 can be formed by laser drilling, for example, or any other suitable technique.

The final implantable device 2 ideally has a width of no more than 3 mm, preferably a width of substantially 2 mm, and a length of no more than 25 mm, preferably a length of no more than 20 mm.

In some exemplary implantable devices 2, such as illustrated in Figure 2, a tracker 15 such as an RFID tracker is also included. The tracker 15 may be used to indicate the location of the animal in which the implantable device 2 has been implanted.

As mentioned previously, using the RFID technology, the sensor 10 can be powered and read wirelessly by the reader 26 in the wearable device 4, and details of this will now be described.

The transponder 14 in the implantable device 2 does not itself have a power source, and so it is a passive transponder 14. Instead, the transponder 14 is powered by electromagnetic energy transmitted from the reader 2 in the wearable device 4. The transponder 14 is therefore configured to receive power transmitted by the wearable device 4 so that the transponder 14 can power the implantable device 2 including the senor 10. Data can be transmitted from the transponder 14 to the reader 26 using electromagnetic induction.

The reader 26 can send an interrogation pulse to the transponder 14 which responds by transmitting a digital data signal identifying the implantable device 2 back to the reader 26.

The energy transmission from the wearable device to the implantable device 2 and the data transmission from the implantable device 2 to the wearable device 4 are both done through inductively coupling the RF coils of the implant antenna 24 with the RF coils of the wearable antenna 28. The implant antenna 24 and the wearable antenna 28 are used as NFC antennas, preferably using a high frequency such as 13.56 MHz.

The optimal number of turns on the RF coil of the implant antenna 24 generally depends on the frequency being used, amongst other factors. Although a higher number of turns of the RF coil provides higher inductance of the implant antenna 24, a higher number of turns also provides a greater inductive reactance which resists rapid changes in current flow i.e., frequency of the oscillation voltage. Generally antenna coils to be used for the high-frequency (HF) RFID range have significantly lower number of turns compared to low-frequency (LF) RFID and are therefore much easier to integrate into the implantable device 2 due to their smaller size. The RFID standard ISO 15693 is suitable which enables compatibility to generally available RFID readers.

The implant antenna 24 comprises a ferrite rod located within the RF coils. In one example antenna, the dimensions of the helical RF coil were set to about 10 mm x 2 mm x 2 mm and a ferrite rod with an exemplary permeability of µ*r* = 40 was inserted in the middle along the winding axis. In other examples, the length of the antenna may be 10 mm long using a 1 mm diameter ferrite rod, or an antenna having a length of 15 mm using a 2 mm diameter ferrite rod. In both these examples, ten windings of 0.1 mm thick copper wire can be wound onto the ferrite rod.

Table 1 shows the impact of the number of turns of the RF coil on the distance at which the transponder 14 can wirelessly communicate with the reader 26. Here a distance of 60 mm was targeted at a low power of 450 µW.

**Table 1**

| **Windings (N)** | **Distance, using 60 mm antenna at low power** |
|---|---|
| 16 | 35 mm |
| 14 | 50 mm |
| 12 | 44 mm |

It was found that transponder antenna coils having a larger diameter achieve a greater reading distance overall, as well as being able to drive a higher current. As can be seen in Table 1, antenna having N = 14 windings performed the best. Generally, the antenna coil windings will be optimised to maximise energy transfer. As demonstrated, the transponder 14 and sensor 10 can be powered wirelessly using inductive HF-RFID principles, even taking into account the size limitations of the implant antenna 24. It has also been found that the reader antenna 28 located in the wearable device 4 provides the highest energy transmission when the RF coil is located on top of the collar or coil inside the collar. In this context, "top" refers to the uppermost in the vertical axis (i.e. top refers to orientation).

As will be appreciated, achievable reading distances and energy transmission differ greatly between the different RFID technologies, which are mainly defined by their operating frequencies and protocols. Due to a combination of the high damping of electromagnetic waves (used in UHF and SHF RFID) by biological tissue, the strict size limitations of the implantable device 2, and the weight limitations of the wearable device 4, only inductive near field coupling in the LF (125 kHz) and HF (13.56 MHz) frequency ranges are employed. Generally, damping effects caused by biological tissue are lower in the LF range, however data transmission rates and energy efficient transmission are better in the HF range.

Looking again at Figure 3, as well as comprising the reader 26, the wearable device 4 also comprises a battery 36 in the form of a rechargeable battery, memory 38 in the form of NOR flash memory, motion sensors 40 for example a 6-axis accelerometer and gyroscope, and a magnetometer 42.

The magnetometer is used to help determine alignment of the wearable antenna 28 with the implant antenna 24. In examples where the wearable device 4 comprises multiple antennae for example 3 wearable antennae, the magnetometer 42 can be used to determine which of the multiple wearable antennae is most optimally aligned with the implant antenna 24 so that the most optimal wearable antenna can be selected to couple with the implantable device so that maximum energy can be transferred from the wearable device 4 to the implantable device 2, and thus energy harvesting by the implantable device is optimised.

The motion sensors 40 can be used to detect movement of the animal 8 and determine its location. The motion sensors 40 are also able to determine the relative position i.e. the relative orientation of the wearable device 4 with respect to the implantable device 2. In this way, the motion sensors 40 can determine whether the reader 26 is in alignment with the implantable device 2. In some examples, the wearable device 4 comprises three antennae. In this way, the wearable antenna that is closest to the implantable device 2 will be activated so that the maximum amount of available RF energy can be sent to the transponder transceiver 22.

The motion sensors 40 are able to detect a lack of movement, for example a lack of change in location, after a minimum limit has passed for example 5 mins. If it is determined that the wearable device 4 has fallen off the animal 8, the wearable device 4 can provide some form of audio or visual indication for example by sounding an alarm.

The memory 38 is used to temporarily store data received from the implantable device 2 until the data can be transferred to an external computing device, for example a remote server. Data can be transferred from the wearable device 4 using a wired or wireless e.g. Wi-Fi connection or LTE connection. When using a wireless connection, data can be transferred from the memory 38 to the external computing device many times a day, for example 8-9 times a day. A power level of 0.45 mW is required which can be harvested effectively with a 20cm distance between the implantable device 2 and the wearable device 4.

The battery 36 can be used to power the wearable device 4. The battery life can be estimated based on how often the sensor 10 gathers data per day as well as how long each measurement takes. The battery life is divided by the total amount of time the reader 26 has to be active per day, excluding the wireless transmission to the external computing device.

Table 2 below table shows the estimated battery life for different power requirements of various components.

**Table 2**

| Reader Output Power | Estimated Power for the RFID Tag [60 mm Diameter Reader coil, 3 cm distance] | Estimated Reader Power Consumption | Estimated Battery Life for an exemplary LiPo battery with 1000 mAh, continuous mode | Estimated Battery Life, for daily active period of one minute |
|---|---|---|---|---|
| 0,2 W | 450 µW | 650 mW [5] | 5,7 h | 342 days |
| 2W | 4,5 mW | 3,31 W | 1,12 h | 67,2 days |
| 4W | 9 mW | 5,98 W | 0,61 h | 36,6 days |

The high-frequency RFID chip used for both the transponder transceiver 22 and the implant antenna 24 operate at a base carrier frequency of 13.56MHz and operate at maximum distances of 10 cm for proximity readers and up to distances of 1m for vicinity readers.

As discussed, the transponder 14 in the implantable device 2 can harvest energy from the wearable device 2 in order to power the passive implantable device 2. The harvested power that can be supplied to the sensor 10 changes depending on the geometric parameters (eg diameter, orientation, etc.) of the transponder 14 and the coils of the reader antenna 28. With the aid of capacitors, power can be stored and the power consumption of the ASIC will be below 0.45 mW to achieve the needed reading distance of about 30 mm even for low power readers.

In summary, the wearable device 4 is able to not only detect the movement and location of the animal 8, but it can also allow energy from the wearable HF RFID chip to be harvested by the transponder 14 to power the sensor 10 in the implantable device 2 and thereby transfer the biomarker data to the NFC transceiver 22.

As will be appreciated, some features and aspects of the invention may be modified without departing from the scope of the invention. For example an alternative method of operating a dielectric sensor could be implemented as will be briefly explained. In this alternative method, open micro-stubs (strip metal line) resonators are embedded in the oscillator of the sensor. The sensing stubs address the change in the dielectric constant by means of propagation constant and characteristic impedance. When the stubs are used the sensing elements, instead of he previously described sensing capacitor, the oscillation frequency and output power are determined by their respective dielectric constants. As a result, for a biological fluid with a unique dielectric constant and a loss tangent pair, the oscillator in the sensor will exhibit a unique combination pair of oscillation frequency and output power, which corresponds to a characteristic behaviour for that particular biomarker in the biological fluid. In other words, the dielectric constant and loss tangent is represented by corresponding oscillation frequency and output power respectively.

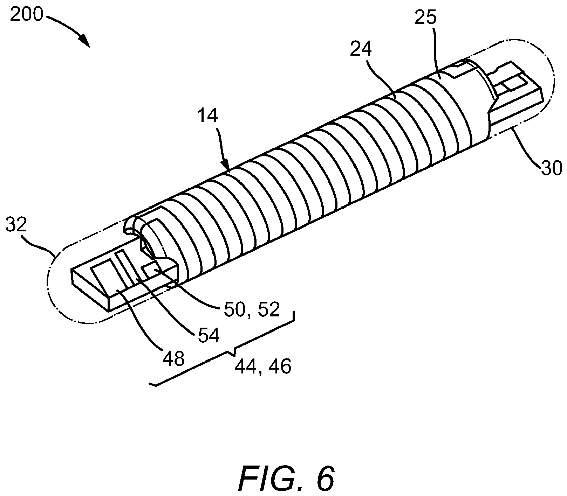

In some implementations, the sensor 10 of an implantable device 200 comprises another sensor 44 in combination with the terahertz sensor described above. The additional sensor 44 is a near-infrared (NIR) sensor 46 (or mid-infrared (MIR) sensor) which includes a photodetector 48 and an emitter 50 as shown in Figure 6. In this example, the emitter 50 takes the form of one or more micro-LEDs 52. The emitter 50 is configured to illuminate the biological fluid at the targeted wavelength. The photodetector 48 is configured to detect a wide range of wavelengths (for example 800nm - 50µm, and more preferably 800nm - 2.5µm for NIR, and 2.5µm -25µm for MIR). This ensures that a wide range of biomarkers can be detected, for example glucose can be detected at wavelengths of around 1100nm. Light from the emitter 50 which is either reflected or absorbed through the biological fluid is then captured by the photodetector 48, which then provides the optical fingerprint to identify the biomarkers in the biological fluid. The use of multiple micro-LEDs 52 as the emitter 50 allows a wide range of wavelengths to be emitted and so multiple biomarker fingerprints can be captured by a single photodetector 48. Generally, an optical barrier 54 is positioned between the photodetector 48 and the emitter 50 to ensure that the photodetector 48 is detecting light than has passed through or been reflected off the biological material, rather than detecting light directly from the emitter 50. Alternatively, the photodetector 48 and the emitter 50 can be located in parallel, i.e. not facing each other, in which case the optical barrier 54 can be omitted. It may also be preferably to apply a coating, e.g. a hydrogel material, to the photodetector 48 and / or emitter 50, where the coating acts as a light waveguide to obtain better signal to noise ratio.

The NIR sensor is complementary to the terahertz sensor previously described and functions together with the terahertz sensor to detect multiple biomarkers. The NIR sensor is able to detect biomarkers that cannot be detected (or would be difficult to detect) using the terahertz sensor, and so the combination of both a NIR sensor and a terahertz sensor means that the implantable device is able to detect a greater number of different biomarkers. The terahertz sensor can detect certain biomarkers and in some applications which require different biomarkers to be detected, the NIR/MIR sensor can be used as an alternate to the terahertz sensor. The NIR/MIR sensor can detect additional biomarkers which can't be detected using the terahertz sensor. The NIR/MIR sensor is powered by NFC in a similar manner as the terahertz sensor. The two sensors are not connected together and do not communicate with each other. However, both sensors are able to communicate with the wearable. The type of biomarkers being detected will determine which sensor is most appropriate to carry out the detection.

As before, and as shown in Figure 6, the implantable device 200 comprises a transponder 14 which includes an implant antenna 24, preferably an NFC antenna in the form of RF coils. The transponder 14 can be embedded on an NFC chip 25 to form an integrated circuit. The transponder 14 functions the same as described previously and so will not be described again. Typically the sensor 44 and transponder 14 are embedded in separate chips connected together by a wire.

As before, the sensor 44 is encapsulated in the single package which is housed within the housing 30, and sealed using the coating 32. The housing and coating are the same as described previously and so will not be described again here. As before, for example in relation to Figure 2, the implantable device 200 may include a tracker 15 such as an RFID tracker which can indicate the location of the animal in which the implantable device 200 has been implanted. In addition, using RFID technology, the sensor 44 can be powered and read wirelessly by the reader 26 in the wearable device 4, and the details of this are the same as described previously so will not be repeated. The final implantable device 200 ideally has a width of no more than 3 mm and a length of no more than 20 mm.

It will be understood that the wearable device 4 and implantable device 2 can be used with any animal and is not limited to use with livestock. For example the wearable device and the implantable device can be used with animals that can be considered as companions, such as pets, including but not limited to cats and dogs.

The wearable device 4 and implantable device 2 can also be used with humans. The implantable device functions in the same way as described previously in relation to animals. That is, the implantable device detects biomarkers in humans in the same way as previously described. The wearable device also functions in the same way as previously described, and the wearable device and implantable device communication with each other in the same manner as previously described. However, for human uses, although the wearable device functions the same way as previously described, the wearable device may be modified for example the wearable device may be a bracelet or ring or any other wearable suitable to be worn by a human instead of a collar, although in some cases the wearable device may be a necklace which may be considered a form of collar.

While the foregoing is directed to exemplary embodiments of the present invention, it will be understood that the present invention is described herein purely by way of example, and modifications of detail can be made within the scope of the invention. Indeed, other and further embodiments of the invention will be apparent to those skilled in the art from consideration of the specification, and may be devised without departing from the basic scope thereof, which is determined by the claims that follow.

## Claims

1. An implantable device for detecting biomarkers in animals, the device comprising:
a sensor comprising:
a pair of electrodes spaced apart from each other, the pair of electrodes having a first capacitance; and
an oscillator configured to oscillate at a first frequency and output a first signal at the first frequency;
wherein the sensor is configured to receive an amount of biological fluid wherein the biological fluid causes the capacitance of the pair of electrodes to change from the first capacitance to a second capacitance;
wherein the oscillator is coupled to the pair of electrodes and arranged such that a change in the capacitance of the pair of electrodes causes a change in oscillation of the oscillator from the first frequency to a second frequency; and
wherein the oscillator is configured to output a second signal at the second frequency, the second signal indicative of a biomarker in the biological fluid.

2. The implantable device according to claim 1 wherein the sensor is a high-frequency terahertz sensor.

3. The implantable device according to any preceding claim further comprising a transponder configured to wirelessly communicate with an external device.

4. The implantable device according to claim 3 wherein the transponder is configured to transmit data corresponding to the second signal to the external device, preferably in response to receiving an indication to transmit data.

5. The implantable device according to any of claims 3 to 4 wherein the transponder is configured to receive power transmitted by the external device so that the transponder can power the implantable device.

6. The implantable device according to any of claims 3 to 5 wherein the sensor and transponder are encapsulated in a package, preferably comprising fan-out wafer-level packaging.

7. The implantable device according to claim 6 wherein the package is housed within a housing, preferably an epoxy mould housing.

8. The implantable device according to claim 7 wherein the housing further comprises a biocompatible coating, preferably a bioglass coating, the coating configured to seal the package within the housing.

9. The implantable device according to any preceding claim further comprising an additional sensor, wherein the additional sensor is a near infrared sensor or mid-infrared sensor.

10. The implantable device according to claim 9 wherein the additional sensor comprises a photodetector and an emitter, and wherein the emitter is configured to emit a light at an amount of biological fluid and the photodetector is configured to detect the light reflected and / or transmitted through the biological fluid in order to determine a biomarker present in the biological fluid.

11. The implantable device according to claim 9 or claim 10 wherein said additional sensor is configured to detect at least one biomarker that is not detectable by said sensor.

12. A system for monitoring the well-being of animals, comprising
an implantable device according to any of claims 1 to 11; and
a wearable device configured to wirelessly communicate with the implantable device.

13. The system according to claim 12 wherein the wearable device is further configured to power the implantable device by generating and transmitting an RF signal which can be received by the transponder of the implantable device, for example using one or more antenna.

14. The system according to claims 12 or 13 wherein the wearable device comprises a least one motion detector configured to detect motion in order to determine a position of the wearable device.

15. A wearable device comprising:
a receiving device configured to receive data from an implant in an animal, wherein the implant is an implantable device according to any of claims 1 to 11, the data indicative of a biomarker present in biological fluid of the animal;
a memory configured to store the data; and
a power generating device configured to wirelessly transmit power to the implant.
